# FASCICULE DE BREVET EUROPEEN

(11) **EP 1 248 679 B1**
(45) Date de publication et mention de la délivrance du brevet: **09.03.2005**
(21) Numéro de dépôt: 01907667.8
(22) Date de dépôt: 22.01.2001
(51) Int. Cl.: B03C 1/01, G01N 33/543, C07K 1/22, C12N 15/10

(54) **PROCEDE D'ISOLEMENT DE PROTEINES OU D'ASSOCIATIONS DE PROTEINES ET D'ACIDES NUCLEIQUES ET COMPLEXES DE PARTICULES ET DE PROTEINES AINSI FORMES**
VERFAHREN ZUR REINIGUNG VON PROTEINEN ODER MISCHUNGEN VON PROTEINEN UND NUKLEINSÄUREN UND SO GESCHAFFENE ZUSAMMESETZUNGEN VON PARTIKELN UND PROTEINEN
METHOD FOR ISOLATING PROTEINS OR PROTEIN AND NUCLEIC ACID ASSOCIATIONS, OR PARTICLE AND PROTEIN COMPLEXES, REAGENT AND USES

(30) Priorité: 21.01.2000 FR 0000862
(43) Date de publication de la demande: 16.10.2002
(73) Titulaire: BIO MERIEUX, 69280 Marcy l'Etoile (FR)
(72) Inventeur: ELAISSARI, Abdelhamid, F-69007 Lyon (FR); MANDRAND, Bernard, F-69100 Villeurbanne (FR); DELAIR, Thierry, F-69700 Echalas (FR); SPENCER, Doran, Eureka, CA 95501 (US); ARKIS, Ahmed, F-69007 Lyon (FR)
(74) Mandataire: Hinterberg, Katherine
(86) Numéro de dépôt international: PCT/FR2001/000205
(87) Numéro de publication internationale: WO 2001/052612

(56) Documents cités:
- WO-A-01/33223
- WO-A-01/33224
- WO-A-89/04373
- WO-A-98/51435
- WO-A-99/07749
- FR-A- 2 762 394
- FR-A- 2 773 416
- CHEMICAL ABSTRACTS, vol. 125, no. 21, 18 novembre 1996 (1996-11-18) Columbus, Ohio, US; abstract no. 269461, RAO, WEI ET AL: "The structure and properties of magnetic micro-beads" XP002152837 & WUHAN GONGYE DAXUE XUEBAO (1996), 18(2), 118-120, 1996,

## Description

Un des problèmes qui se rencontre de manière récurrente concerne l'extraction, la purification, la concentration et la conservation de matériel biologique en vue d'une utilisation ultérieure.

Une des méthodes de purification de protéines utilisée est la purification par précipitation en utilisant des agents de précipitation, tels que le sulfate d'ammonium ou des polyacides acryliques (H. Morawetz and W. L. Hughes, J. Phys. Chem., 56, 64-69 (1952)).

Une autre méthode concerne l'extraction de matériel biologique au moyen de suspensions colloïdales. On peut citer la demande de brevet WO-A-98/01482 de Krupey qui décrit une extraction de protéines dans un milieu aqueux en utilisant des colloïdes à base de copolymères d'anhydride maléique réticulés. Après complexation des protéines sur les colloïdes, les complexes sont extraits du milieu par centrifugation. Une telle méthode présente l'inconvénient de nécessiter une étape de centrifugation relativement délicate et faisant appel à un appareillage lourd, coûteux, qui de plus n'est pas toujours facilement accessible.

Une solution possible est d'utiliser des colloïdes d'oxydes de fer magnétiques, tels que décrits par Rao VC et al. (Appl. Environ. Microbiol., 1981, Sep, 42 (3) : 421-426). Toutefois, il est connu que les oxydes de fer sont incompatibles avec les techniques d'amplification enzymatique d'acides nucléiques parce qu'ils inhibent les enzymes.

Pour résoudre ce problème, il a été proposé (demande de brevet WO-A-99/35500) de recouvrir des particules magnétiques d'un polymère hydrophile cationique ou anionique, qui masque les oxydes de fer et qui permet ainsi de lever l'inhibition de la réaction d'amplification enzymatique, après une étape d'extraction des acides nucléiques. Ce polymère est un polymère thermosensible qui lorsqu'il est chauffé à une température supérieure à 32°C devient hydrophobe et peut fixer des protéines par des interactions hydrophobes. Cependant, les interactions hydrophobes sont dénaturantes pour un grand nombre de protéines.

Les présents inventeurs ont maintenant trouvé des particules colloïdales magnétiques qui pallient tous les inconvénients précités et qui de plus sont ubiquitaires, en ce sens qu'elles permettent l'isolement de protéines et d'associations de protéines et d'acides nucléiques à partir d'un milieu complexe de façon simple, efficace, rapide, ne nécessitant pas d'appareillage coûteux et compatibles avec les techniques d'amplification enzymatique. Elles n'impliquent pas d'appareillage lourd, ni beaucoup de manipulations. De telles particules sont donc utilisables, indépendamment de l'environnement dans lequel elles se trouvent et permettent, entre autres, l'extraction d'associations de protéines et d'acides nucléiques, mais également l'extraction de protéines.

Par protéines, on entend, les holoprotéines et les hétéroprotéines ou leurs fragments, c'est à dire, les lipoprotéines, les glycoprotéines, les hémoprotéines, les phosphoprotéines, les flavoprotéines, les métalloprotéines, les polypeptides, les antigènes, les immunogènes, les anticorps, les enzymes.

On entend notamment par association de protéines et d'acides nucléiques, des structures complexes nucléoprotéiques comme les chromosomes, les histones, et aussi les virus, les bactéries, les champignons.

De telles particules sont particulièrement intéressantes dans les régions où les virus à fièvres hémorragiques sont endémiques.

En particulier, les particules de l'invention peuvent être transportées, sans étape de lyophilisation, ni de congélation, ce qui présente des avantages incontestables, notamment en terme de sécurité lors du transport d'échantillons infectieux. Une épidémie récente au Congo a montré les difficultés de terrain qui peuvent être rencontrées, quand des échantillons d'origine humaine qui posaient des risques très importants de contamination ont été transportés en Afrique du Sud pour un diagnostic (Organisation Mondiale de la Santé, 1999 :
http ://www.who.int/emc/outbreak_news/n1999/may/n28may1999.html).

De plus, les particules colloïdales de l'invention permettent, si souhaité, d'infecter des cellules mises en culture par un agent infectieux, ainsi isolé, purifié, concentré et éventuellement transporté. Aussi, les particules colloïdales de l'invention trouvent de nombreuses applications et utilisations.

Dans le cas des virus, par exemple, une étape de lyse à l'aide d'agents chaotropiques, de la chaleur ou d'autres moyens est indispensable préalablement à la phase d'amplification enzymatique, ce qui requiert d'utiliser des colloïdes stables dans des conditions de forte stringence. La méthode de préparation de ces colloïdes stables est incorporée dans les exemples de la présente demande de brevet à titre d'illustration.

Ainsi, la présente invention concerne un procédé d'isolement de protéines et/ou d'une association de protéines et d'acides nucléiques, dans un échantillon, caractérisé en ce que :
- on met en contact ledit échantillon et des particules colloïdales magnétiques comprenant un coeur et une enveloppe dans lesquelles :
   le coeur est magnétique et est enrobé par au moins un polymère comprenant des groupements fonctionnels X choisis parmi les groupements amine, hydroxyle, thiol, aldéhyde, ester, anhydride, chlorure d'acide, carbonate, carbamate, isocyanate et isothiocyanate ou leurs mélanges, dont au moins une fraction a réagi avec d'autres groupements fonctionnels de l'enveloppe, et
   l'enveloppe est constituée d'un polymère portant des groupements fonctionnels ionisables, Z et Z', identiques ou différents, choisis parmi les groupements amine, acide carboxylique, ester, anhydride, aldéhyde, thiol, disulfure, α-halogénocarbonyle, acide sulfonique, maléimide, isocyanate, et isothiocyanate, qui ont réagi partiellement avec les groupements fonctionnels X du coeur ;
   pour constituer un mélange,
- on incube ledit mélange sous des conditions prédéterminées, et
- on sépare du mélange les protéines et/ou les associations de protéines et d'acides nucléiques complexées sur lesdites particules colloïdales par application d'un champ magnétique.

En particulier, le coeur magnétique est solide et constitué de particules d'oxydes métalliques ou il consiste en une émulsion comprenant des particules d'oxydes métalliques, lesdites particules d'oxydes métalliques ou ladite émulsion de particules d'oxydes métalliques.

Par réaction partielle des groupements fonctionnels Z, Z' avec X, on comprend que des groupements Z et/ou Z' doivent rester disponibles. La totalité ou au moins une partie de ces groupements Z et/ou Z libres réagiront par interaction ionique avec les groupements ioniques des protéines et/ou associations de protéines et d'acides nucléiques.

Le tableau 1 ci-dessous résume la complémentarité entre les différents groupements fonctionnels X, Z et Z'.

**Tableau 1**

| X | Z | Z' |
|---|---|---|
| ester, anhydride, chlorure d'acide, carbonate, carbamate, isocyanate, isothiocyanate | Amine | amine, thiol, acide carboxylique, ester, acide sulfonique |
| Aldéhyde | Amine | amine, thiol, acide carboxylique, ester, acide sulfonique |
| Amine, hydroxyle | Acide carboxylique, ester, anhydride, aldéhyde, isocyanate, isothiocyanate | acide carboxylique, acide sulfonique |
| Thiol | thiol, disulfure, α-halogénocarbonyle, maléimide | amine, acide carboxylique, ester, anhydride, acide sulfonique |

Le coeur des particules colloïdales comprend au moins un polymère organique choisi parmi au moins un homopolymère ou un copolymère ou leurs mélanges, issu de la polymérisation d'au moins un monomère choisi parmi les monomères d'acrylamide et d'acrylate, en particulier les N-alkylacrylamide et N-N-dialkylacrylamide, tels que le N-isopropylacrylamide, le N-méthylacrylamide, le N-éthylméthacrylamide, le N-n-propylacrylamide, le N-n-propylméthacrylamide, le N-isopropylméthacrylamide, le N-cyclopropylacrylamide, le N,N-diéthylacrylamide, le N-méthyl-N-isopropylacrylamide, le N-méthyl-N-n-propylacrylamide ; les acrylates et les méthacrylates d'alkyle dans lesquels le groupement alkyle comprend de 3 à 20 atomes de carbone ; le styrène, le méthylstyrène, l'éthylstyrène, le tertio-butyl-styrène, le chlorométhylstyrène vinyltoluène ; leurs dérivés et les copolymères de ces monomères entre eux et/ou avec d'autres comonomères, et des particules d'oxydes métalliques choisies parmi les particules d'oxydes métalliques, de fer, de titane, de cobalt, de zinc, de cuivre, de manganèse, de nickel ; la magnétite ; l'hématite, les ferrites, telles que les ferrites de manganèse, nickel, manganèse-zinc; les alliages de cobalt, nickel.

Le polymère de l'enveloppe est choisi parmi au moins un homopolymère ou copolymère hydrophile choisi parmi les homopolymères ou copolymères :
- issus de la polymérisation d'au moins un monomère choisi parmi les monomères dérivés de l'acrylamide ou de méthacrylamide ; l'acide acrylique, l'acide méthacrylique ; les dérivés acrylates et méthacrylates ; l'allylamine; les dérivés styréniques ; à la condition s'il s'agit d'un homopolymère que celui ci comporte des groupements fonctionnels ionisables en particulier les copolymères ou homoplymères de l'anhydride maléique et les homopolymères ou copolymères de l'acryloxysuccinimide,
- les polysaccharides, tels que le chitosane et le polyacide galacturonique,
- les polypeptides, tels que la polylysine et la polyarginine,
- la polyéthylèneimine linéaire ou ramifiée, et
- les dendrimères ;
de préférence le poly(anhydride maléique vinyl éther), le poly(N-vinylmorpholine-N-acryloxysuccinimide) ou le poly(N-vinylpyrrolidone-N-acryloxysuccinimide).

Le mélange est soumis à incubation à une température comprise entre 15 et 60°C, de préférence entre 20 et 35°C, pendant un temps d'incubation compris entre 5 et 60 minutes, de préférence 10 minutes.

L'échantillon peut être un échantillon biologique, par exemple un prélèvement, tel qu'un prélèvement tissulaire, de sang total ou de sérum ou un surnageant de culture, ou bien un prélèvement agroalimentaire, comprenant des protéines et/ou des associations de protéines et d'acides nucléiques, en particulier un virus, une bactérie, une levure, une cellule, éventuellement en mélange. Par prélèvement agroalimentaire, on entend tout échantillon agroalimentaire susceptible d'être ou d'avoir été infecté par un agent infectieux.

L'invention concerne également un procédé d'extraction de protéines et/ou d'une association de protéines et d'acides nucléiques, selon lequel on isole d'un échantillon, par exemple un prélèvement agroalimentaire ou un échantillon biologique tel qu'un prélèvement ou un surnageant de culture, un virus et/ou une bactérie et/ou une levure et/ou, une cellule ou un mélange de ces derniers selon le procédé décrit précédemment et, on soumet, si nécessaire, lesdits virus, bactérie, levure, cellule ou leur mélange à une étape de relargage et/ou de dénaturation partielle ou totale pour l'extraction desdites protéines et/ou desdits acides nucléiques.

L'invention concerne aussi un procédé d'identification et/ou de détection et/ou de quantification de protéines et/ou d'une association de protéines et d'acides nucléiques et/ou d'acides nucléiques, selon lequel on isole d'un échantillon, par exemple d'un prélèvement ou d'un surnageant de culture, un virus et/ou une bactérie et/ou une levure et/ou une cellule ou un mélange de ces derniers selon le procédé décrit précédemment, on soumet, si nécessaire, lesdits virus et/ou bactérie et/ou levure et/ou cellule ou leur mélange à une étape de relargage et/ou de dénaturation partielle ou totale pour l'extraction desdites protéines et/ou desdits acides nucléiques, et on identifie et/ou détecte et/ou quantifie lesdites protéines par immunodosage et/ou lesdits acides nucléiques par amplification de ces derniers et/ou par hybridation d'au moins une sonde nucléotidique spécifique desdits acides nucléiques à identifier et/ou détecter et/ou quantifier.

Par immunodosage, on entend la détection et/ou la quantification d'au moins une protéine ou d'au moins un antigène par la mise en évidence d'un complexe protéine ou antigène/anticorps, en particulier par les techniques dites de Western blot, de compétition ou sandwich.

Par amplification, on entend toutes les techniques appropriées pour l'amplification d'ADN ou d'ARN, en particulier la PCR, la RT-PCR, le NASBA, la TMA. Il peut s'agir entre autre d'une amplification quantitative. Par ailleurs, les acides nucléiques à identifier et/ou détecter et/ou quantifier peuvent l'être par hybridation d'au moins une sonde nucléotidique marquée par tout marqueur approprié, de préférence par hybridation de la cible à une sonde de capture et à une sonde de détection. On peut citer à titre d'exemple non limitatif les techniques bien connues de l'homme de l'art Southern Blot, Northern Blot et la technique ELOSA (Enzyme Linked Oligosorbent Assay) (Katz JB et al., Am. J. Vet. Res., 1993 Dec ; 54 (12) :2021-6 et François Mallet et al., Journal of Clinical Microbiology, June 1993, p1444-1449).

Les protéines sont des protéines de surface ou intracellulaires d'un virus, d'une bactérie, d'une levure ou d'une cellule et les acides nucléiques sont de l'ADN et/ou de l'ARN. Les protéines sont identifiées et/ou détectées et/ou quantifiées (i) soit directement, sans étape de relargage et/ou de dénaturation, à partir desdits virus et/ou bactérie et/ou levure et/ou cellule isolés ou dans un surnageant de culture, (ii) soit indirectement; après une étape de relargage et/ou de dénaturation partielle ou totale réalisée, par exemple, par modification de pH (abaissement ou élévation du pH par addition d'une solution acide ou basique). Les acides nucléiques sont identifiés et/ou détectés et/ou quantifiés après une étape de relargage et/ou de dénaturation partielle ou totale desdits virus et/ou bactérie et/ou levure et/ou cellule isolés réalisée, par exemple, par un traitement chimique et/ou physique. A titre d'exemple, un traitement chimique inclut l'utilisation d'agents tensioactifs, comme le SDS, LLS, l'utilisation d'agents chaotropiques comme le thiocyanate de guanidium, l'utilisation d'enzymes comme les protéases (protéinase K) ou autres agents appropriés. A titre d'exemple, un traitement physique comprend la sonication, le chauffage, les ultra sons, l'agitation mécanique (avec par exemple des billes rigides de type verre) ou analogues.

L'invention concerne également un procédé pour la mise en culture d'un virus et/ou d'une bactérie et/ou d'une levure et/ou de cellules selon lequel on isole à partir d'un échantillon, par exemple un prélèvement ou un surnageant de culture, ledit virus et/ou ladite bactérie et/ou ladite levure et/ou lesdites cellules selon le procédé décrit précédemment, on met en culture, dans un milieu de culture et sous des conditions appropriés, lesdits virus et/ou bactérie et/ou levure et/ou cellules ainsi isolés, et si souhaité, on identifie et/ou détecte et/ou quantifie lesdits virus et/ou bactérie et/ou levure et/ou cellules, selon des techniques bien connues de l'homme du métier.

Un autre objet de l'invention est un procédé de préparation d'un échantillon biologique, tel qu'un prélèvement ou un surnageant de culture, ou un prélèvement agroalimentaire, selon lequel on isole de l'échantillon des protéines et/ou des associations de protéines et d'acides nucléiques selon le procédé ci-dessus et/ou on réalise une culture selon un procédé tel que décrit précédemment.

L'invention concerne encore un complexe formé de particules colloïdales magnétiques et de protéines et/ou d'une association de protéines et d'acides nucléiques, les protéines et/ou acides nucléiques étant immobilisées par interactions électrostatiques et/ou par adsorption. Les particules colloïdales comprennent un coeur et une enveloppe dans lesquelles :
le coeur est magnétique et est enrobé par au moins un polymère comprenant des groupements fonctionnels X choisis parmi les groupements amine, hydroxyle, thiol, aldéhyde, ester, anhydride, chlorure d'acide, carbonate, carbamate, isocyanate et isothiocyanate ou leurs mélanges, dont au moins une fraction a réagi avec d'autres groupements fonctionnels de l'enveloppe, et
l'enveloppe est constitué d'un polymère portant des groupements fonctionnels ionisables Z et Z', identiques ou différents, choisis parmi les groupements amine, acide carboxylique, ester, anhydride, aldéhyde, thiol, disulfure, α-halogénocarbonyle, acide sulfonique, maléimide, isocyanate, et isothiocyanate, dont au moins une partie a réagi avec les groupements fonctionnels X du coeur ; et
les protéines et/ou associations de protéines et d'acides nucléiques sont issues d'un échantillon biologique, par exemple d'un prélèvement, tel qu'un prélèvement tissulaire, de sang total, de sérum, ou d'un surnageant de culture, ou d'un prélèvement agroalimentaire.

En particulier, le coeur des particules colloïdales est solide et constitué de particules d'oxydes métalliques ou essentiellement solide et constitué d'une émulsion comprenant des particules d'oxydes métalliques.

De préférence, le prélèvement ou le surnageant de culture sont infectés par au moins un virus, une bactérie, une levure ou leur mélange.

Le coeur comprend au moins un polymère organique choisi parmi au moins un homopolymère ou un copolymère ou leurs mélanges, issu de la polymérisation d'au moins un monomère choisi parmi les monomères d'acrylamide et d'acrylate, en particulier les N-alkylacrylamide et N,N-dialkylacrylamide, tels que le N-isopropylacrylamide, le N-méthylacrylamide, le N-éthylméthacrylamide, le N-n-propylacrylamide, le N-n-propylméthacrylamide, le N-isopropylméthacrylamide, le N-cyclopropylacrylamide, le N,N-diéthylacrylamide, le N-méthyl-N-isopropylacrylamide, le N-méthyl-N-n-propylacryiamide ; les acrylates et les méthacrylates d'alkyle dans lesquels le groupement alkyle comprend de 3 à 20 atomes de carbone ; le styrène, le méthylstyrène, l'éthylstyrène, le tertio-butyl-styrène, le chlorométhylstyrène vinyltoluène ; leurs dérivés et les copolymères de ces monomères entre eux et/ou avec d'autres comonomères, et des particules d'oxydes métalliques choisies parmi les particules d'oxydes métalliques, de fer, de titane, de cobalt, de zinc, de cuivre, de manganèse, de nickel ; la magnétite ; l'hématite, les ferrites, telles que les ferrites de manganèse, nickel, manganèse-zinc; les alliages de cobalt, nickel.

Le polymère de l'enveloppe est choisi parmi au moins un homopolymère ou copolymère hydrophile choisi parmi les homopolymères ou copolymères :
- issus de la polymérisation d'au moins un monomère choisi parmi les monomères dérivés de l'acrylamide ou de méthacrylamide ; l'acide acrylique, l'acide méthacrylique ; les dérivés acrylates et méthacrylates ; l'allylamine ; les dérivés styréniques ; à la condition s'il s'agit d'un homopolymère que celui ci comporte des groupements fonctionnels ionisables ; en particulier les copolymères ou homoplymères de l'anhydride maléique et les homopolymères ou copolymères de l'acryloxysuccinimide,
- les polysaccharides, tels que le chitosane et le polyacide galacturonique,
- les polypeptides, tels que la polylysine et la polyarginine,
- la polyéthylèneimine linéaire ou ramifiée, et
- les dendrimères ;
en particulier, le poly(anhydride maléique vinyl éther) le poly(N-vinylmorpholine-N-acryloxysuccinimide) ou le poly(N-vinylpyrrolidone-N-acryloxysuccinimide).

Ce complexe est utilisé pour le transfert et/ou le transport et/ou le stockage d'agents infectieux, en particulier de virus et/ou bactérie et/ou levure, à l'état sec ou dans un tampon approprié.

L'invention concerne encore un réactif pour l'extraction et/ou l'identification et/ou la détection et/ou la quantification de protéines et/ou d'associations de protéines et d'acides nucléiques, caractérisé en ce qu'il comprend, entre autres :
un complexe tel que défini ci-dessus ou des particules colloïdales telle que définies ci-dessus permettant d'obtenir un complexe de l'invention, et
éventuellement au moins un moyen pour l'identification et/ou la détection et/ou la quantification desdites protéines ou desdits acides nucléiques, en particulier au moins un anticorps monoclonal ou polyclonal pour l'identification et/ou la détection et/ou la quantification d'au moins une protéine, au moins une amorce, de préférence au moins deux amorces et/ou au moins une sonde nucléotidique, de préférence au moins deux sondes nucléotidiques spécifiques d'au moins un acide nucléique pour son identification et/ou sa détection et/ou sa quantification.

Enfin, l'invention concerne une composition vaccinale qui comprend, à titre de principe actif, au moins un complexe de l'invention tel que défini précédemment, éventuellement associé à un véhicule et/ou un excipient et/ou un adjuvant et/ou un diluant pharmaceutiquement acceptable.

Par " véhicule pharmaceutiquement acceptable " on entend les supports et véhicules administrables à l'être humain ou à un animal, tels que décrits par exemple dans Remington's Pharmaceutical Sciences 16^{th} ed., Mack Publishing Co. Le véhicule pharmaceutiquement acceptable est de préférence isotonique, hypotonique ou présente une faible hypertonicité et a une force ionique relativement basse. Les définitions des excipients et adjuvants pharmaceutiquement acceptables sont également données dans Remington's Pharmaceutical Sciences précité.

Un autre objet de l'invention est un véhicule pharmaceutiquement acceptable pour une composition vaccinale, consistant en une particule magnétique répondant à la définition de la présente invention.

### Exemple 1: Obtention de latex magnétiques carboxyliques.

Le polymère poly(anhydride maléique méthyl vinyl éther) (AMVE) est solubilisé dans du diméthylsulfoxyde (DMSO) anhydre (2g/l). 50 µl de cette solution d'AMVE sont dilués dans 1 ml de tampon phosphate (pH 6,8; 10 mM) puis incubés 10 minutes à 37°C. On mélange ensuite 1 ml d'une dispersion de latex magnétiques aminé (0,5% dans l'eau 1x la concentration micellaire critique (CMC) du Triton X-405) fabriqué, par exemple selon le protocole décrit dans la demande de brevet PCT WO 99/35500, avec 125 µl du mélange (AMVE-DMSO-tampon) préalablement préparé. Le mélange est incubé à 37°C pendant 3 heures. Les particules peuvent ensuite être solubilisées telles quelles ou après une étape de purification, par exemple par centrifugation.

### Exemple 2: Extraction sélective de particules virales à partir de sérum, de plasma ou de surnageant de culture.

### - Purification des particules virales :

A 5 µl de latex magnétique fonctionnalisé par le copolymère AMVE, comme décrit dans l'exemple 1, à 3% de taux de solide, sont ajoutés directement de 100 à 150 µl de sérum positif, c'est à dire comprenant des particules virales. L'échantillon n'est pas tamponné.

Le mélange sérum-latex est homogénéisé au vortex pendant 10 secondes puis laissé à température ambiante pendant 10 minutes pour la fixation des particules virales par le latex magnétique. Cette étape de capture est suivie par la récupération du complexe latex-particules virales, sous forme de culot débarrassé du surnageant, par simple aimantation pendant 30 secondes.

Le culot peut être lavé ou non une fois par 100 µl d'HEPES, pH 6,5, aimanté comme auparavant puis dispersé dans 50 µl d'eau stérile.

### - Extraction des acides nucléiques après lyse des particules virales par la chaleur :

Le complexe latex-particules virales dispersé dans de l'eau est incubé à 95°C pendant 5 minutes afin de lyser les particules virales. Les acides nucléiques sont récupérés dans le surnageant débarrassé du latex par aimantation.

### Exemple 3: capture du virus de l'hépatite G par un latex magnétique, fonctionnalisé de l'invention.

- Purification des particules virales

A 5 µl du latex magnétique fonctionnalisé par le copolymère AMVE, comme décrit à l'exemple 1, à 3% de taux de solide, sont ajoutés des volumes croissants, respectivement de 25 µl et 50 µl de sérum humain non dilué, positif pour le virus de l'hépatite G, dont le titre viral est inconnu.

Le mélange sérum-latex est homogénéisé au vortex pendant 10 secondes puis laissé à température ambiante pendant 10 minutes pour la fixation des particules virales par le latex magnétique. Cette étape de capture est suivie par la récupération du complexe latex-particules virales, sous forme de culot débarrassé du surnageant, par simple aimantation pendant 5 minutes.

Le culot est ensuite lavé une fois par 50 µl d'HEPES-Tween (0,25/∞, pH6.5) aimanté comme auparavant, puis dispersé dans 50 µl d'eau stérile.

### - Extraction des acides nucléiques :

Une étape de lyse des particules virales est ensuite effectuée à l'aide du kit QIAmp Viral RNA Mini Kit (nom commercial), commercialisé par la société QIAGEN, pour la libération des acides nucléiques viraux.

### - Amplification des acides nucléiques :

Les acides nucléiques viraux sont ensuite amplifiés par RT-PCR en une étape (1) (Life Technologies) et PCR nichée (2) en utilisant les couples d'amorces suivantes (Smith DB et al., Discrimination of hepatitis G virus/GBV-C geographical variants by analysis of the 5' non-coding region, J. Gen. Virol., 1997, 78 : 1533-1542) :

(1) : amorce 1 : 5′ AGG TGT GGA TGG GTG ATG 3′
amorce 2 : 5′ ATG CCA CCC GCC CTC ACC CGA 3′

(2) :   amorce 1 : 5′  TTG GTA GGT CGT AAA TCC CGG 3′
            amorce 2 : 5′  CGG AGC TGG GTG GCC CCA TGC ATT 3′

Les résultats, après passage sur un gel d'agarose 1 % et coloration au bromure d'éthidium, montrent une bande de taille attendue de 343 paires de bases, après la deuxième PCR. Le marqueur de poids moléculaire utilisé comme référence est le marqueur Smartladder (nom commercial) (multiples de 200 paires de bases) de la société Eurogentec. Ces résultats confirment que le latex fonctionnalisé de l'invention est capable de capturer les particules virales de l'hépatite G et de les retenir lors des étapes de lavage.

Par ailleurs, 10 µl de sérum humain positif pour HGV, de titre inconnu, ont été dilués dans 3 ml de sérum humain négatif et mis en présence de 10 µl du latex de l'invention, obtenu selon l'exemple 1. Les étapes de purification des particules virales, d'extraction des acides nucléiques et d'amplification ont été réalisées comme décrit ci-dessus.

Les résultats obtenus montrent, après passage des produits d'amplification sur gel d'agarose 1 % et coloration au bromure d'éthidium, la bande de taille attendue de 343 paires de bases, ce qui confirme la capacité du latex de l'invention à capturer et retenir les particules virales à partir d'un échantillon dilué de grand volume.

### Exemple 4: capture du virus de l'hépatite C par un latex magnétique fonctionnalisé de l'invention.

### - Purification des particules virales :

A 5 µl du latex magnétique fonctionnalisé par le copolymère AMVE, comme décrit à l'exemple 1, à 3% de taux de solide, sont ajoutés des volumes 50 µl d'un sérum humain non dilué, positif pour le virus de l'hépatite C, dont le titre viral est inconnu.

Le mélange sérum-latex est homogénéisé au vortex pendant 10 secondes puis laissé à température ambiante pendant 10 minutes pour la fixation des particules virales par le latex magnétique. Cette étape de capture est suivie par la récupération du complexe latex-particules virales, sous forme de culot débarrassé du surnageant, par simple aimantation pendant 5 minutes.

Le culot est ensuite lavé une fois par 50 µl d'HEPES-Tween (0,25/∞, pH6.5) aimanté comme auparavant, puis dispersé dans 50 µl d'eau stérile.

### - Extraction des acides nucléiques :

Une étape d'extraction des acides nucléiques est ensuite effectuée à l'aide du kit QIAmp Viral RNA Mini Kit (nom commercial), commercialisé par la société QIAGEN, pour la libération des acides nucléiques viraux.

### - Amplification des acides nucléiques :

Les acides nucléiques viraux sont ensuite amplifiés par RT-PCR en une étape (1) (Life Technologies), suivie d'une PCR semi nichée (2) en utilisant les couples d'amorces suivants ( Li JS et al., Identification of the third major genotype of hepatitis C in France, BBRC, 1994, 3 : 1474-1481) :

(1) amorce 1 : 5′ CCT GTG AGG AAC TAC TGT CTT CAC GCA 3′
amorce 2 : 5′ ACT CGC AAG CAC CCT ATC AGG CAG TAC 3′

(2) amorce 1 : 5′ AAG CGT CTA GCC ATG GCG TTA GTA T 3′
amorce 2 : 5′ ACT CGC AAG CAC CCT ATC AGG CAG TAC 3′

Les résultats, après passage sur un gel d'agarose 1% et coloration au bromure d'éthidium, montrent une bande de taille attendue de 240 paires de bases, après la deuxième PCR. Le marqueur de poids moléculaire utilisé comme référence est le marqueur Smartladder (nom commercial) (multiples de 200 paires de bases) de la société Eurogentec. Ces résultats confirment que le latex fonctionnalisé de l'invention est capable de capturer les particules virales de l'hépatite C et de les retenir lors des étapes de lavage.

### Exemple 5 détection du virus de la rougeole.

A partir d'échantillons viraux préalablement titrés, une extraction des acides nucléiques a été réalisée à l'aide du kit commercialisé par la société QIAGEN (kit QIAmp. Viral RNA Mini Kit (nom commercial)), selon le protocole du fournisseur. Les acides nucléiques ont ensuite été amplifiés par RT-PCR à l'aide du kit TITAN (nom commercial - Roche) en présence d'1 µl d'inhibiteur des ARNases (RNAsine, nom commercial - Promega) ou par PCR nichée.
- Titrage des échantillons viraux (souche Edmonston) : des dilutions successives du virus ont été préparées ( de 10⁻¹ à 10⁻⁷). 200 µl de ces dilutions ont été distribuées dans les 6 puits d'une plaque contenant des cellules Vero (cellules de rein de singe vert) cultivées dans un milieu 199 contenant du glutamax (Gibco-BRL), 1 % d'antibiotiques (Gibco-BRL) et 1 % de sérum de veau foetal. Les cellules Vero sont confluentes à 80%. L'incubation a été réalisée pendant une heure à 37°C sous CO₂ (5%). 200 µl du milieu contenant le virus ont été éliminés et remplacés par 200 µl de milieu exempt de virus. L'incubation a été réalisée dans les mêmes conditions pendant 72 à 96 heures. Les cellules sont ensuite examinées et les plages de lyse sont visualisées et comptées. 60 ml de culture au titre de 5,5 10⁶ pfu/ml (unité formant une plage de lyse/ml) ont été obtenus, aliquotés et stockés à - 80°C.
- Extraction des acides nucléiques: les acides nucléiques sont extraits de la culture (5,5 10³ pfu/µl) à l'aide du kit de la société QIAGEN précité.
- Amplifications : les acides nucléiques extraits sont amplifiés par RT-PCR en utilisant le couple d'amorces de PCR suivant : amorce 1 : 5′ CCC ATT ACA TCA GGA TCC GG 3′ amorce 2 : 5′ TTG GTC CGC CTC ATC CTC CA 3′ éventuellement suivie d'une PCR nichée en utilisant le couple d'amorces suivant :

amorce 1 : 5′ GGT ACC TCT TGA TGC GAA GG 3′

amorce 2 : 5' GGC CAC ACT TTT AAG GAG CTT A 3'

Les produits d'amplification sont ensuite passés sur gel d'agarose 2% (TBE + Gelstar). Le marqueur de poids moléculaire utilisé comme référence est le marqueur Smartladder (nom commercial) (multiples de 100 paires de bases) de la société Eurogentec. Les résultats montrent une bande de taille attendue de 384 paires de bases pour la RT-PCR et de 309 paires de bases pour la PCR nichée, ce qui confirme la bonne extraction des ARNs. viraux et l'efficacité aussi bien de la RT-PCR que de la PCR nichée.

### Exemple 6: capture du virus de la rougeole par le latex fonctionnalisé de l'invention.

Après l'obtention d'échantillons viraux titrés, comme décrit dans l'exemple 8, la détermination quantitative du niveau de capture et de détection viral par le latex de l'exemple 1 a été réalisée. Une gamme de titre viraux a été effectuée en diluant la solution mère dans du plasma humain négatif. Les dilutions sont effectuées au 1/10 dans un volume final de 1000 µl. La quantité de latex est de 35 µl (945 µg) par échantillon. Après capture, une extraction des ARNs viraux est effectuée à l'aide du kit QIAGEN précité. Les ARNs viraux extraits sont ensuite amplifiés par RT- PCR suivie d'une PCR nichée en utilisant les couples d'amorces décrits dans l'exemple 5. Les produits d'amplification sont ensuite passés sur un gel d'agarose 2% (TBE + Gelstar). Un marqueur de poids moléculaire (multiple de 200 paires de bases) (Smartladder - Eurogentec) est utilisé comme référence. Le témoin positif est représenté par les ARNs totaux extraits et le témoin négatif est constitué par du plasma négatif. Les résultats sont représentés dans le tableau 2 ci-dessous.

**Tableau 2**

| témoin négatif | témoin positif | dilutions en pfu (unité formant une plage de lyse) | | | | | | | |
|---|---|---|---|---|---|---|---|---|---|
| Plasma négatif | ARNs totaux | 10⁶ | 10⁵ | 10⁴ | 10³ | 10² | 10 | 1 | 10⁻¹ |
| - | + | + | + | + | + | + | + | + | - |

Le symbole + signifie la présence du produit d'amplification de taille attendue (309 paires de bases) et le symbole - signifie une absence de produit d'amplification.

Les résultats montrent que la PCR nichée donne un produit spécifique d'amplification jusqu'à 1 pfu par tube (100 µl). L'absence de produit d'amplification à la dilution 0,1 pfu indique qu'il n'y a pas de détection de particules virales non infectieuses.

### Exemple 7 : étude de la stabilité du complexe latex/virus de la rougeole.

Afin d'étudier la stabilité des complexes latex/virus, par rapport à la détection des ARNs viraux par RT-PCR, les complexes ont été révélés au cours du temps. Plusieurs titres infectieux ont été testés, ainsi que des traitements différents des échantillons, dans l'optique de préserver les échantillons tout en réduisant le nombre de manipulations. Les échantillons ont été conservés sous une hotte à flux laminaire, à température ambiante.

Les complexes ayant subi ou non des traitements ont été testés respectivement aux jours 0, 1, 2, 3, 7 et 14 :
Traitement A : échantillon plasmatique contenant du virus et le latex, qui sert de témoin de base (sans traitement).
Traitement B : complexe latex/virus, après élimination du plasma, sans lavage et laissé à sec.
Traitement C : complexe latex/virus, après élimination du plasma et une étape de lavage et laissé à sec.
Traitement D : complexe latex/virus, après élimination du plasma et une étape de lavage et conservé en présence du tampon de lavage.

Les titres infectieux des échantillons testés sont respectivement de 50, 500 et 5 000 pfu par échantillon pour chaque traitement.

Les complexes sont ensuite lysés par la chaleur pour libérer les acides nucléiques et les acides nucléiques sont passés sur des gels d'agarose 2% (TBE + Gelstar) avec un marqueur de poids moléculaire utilisé comme référence (Smartladder - Eurogentec).

Les résultats, dans les conditions de l'expérience, montrent que le traitement B ne permet pas de conserver les particules virales de manière stable. Mais, il convient de noter que si une étape de lavage est effectuée avant l'étape d'extraction des acides nucléiques, le latex de l'invention conserve les particules de manière stable, comme l'ont montré les inventeurs dans une autre expérience. Par contre, les complexes C et D qui subissent une étape de lavage permettent de conserver les échantillons, sans que les ARNs soient dégradés, comme démontré par RT-PCR. Ceci signifie qu'une étape de lavage dans un tampon est nécessaire pour la conservation de l'échantillon capturé, que ce dernier soit conservé dans le tampon ou à sec, si l'on veut pratiquer l'étape de lyse dans le tube d'extraction, mais un moyen d'éviter cette étape de lavage est de changer de tube. Bien entendu, une conservation à sec est plus avantageuse puisqu'elle permet de transporter les échantillons capturés du lieu de prélèvement à un site d'analyse en évitant tout risque de contamination et de dissémination.

### Exemple 8 : capture du virus de la rougeole à partir de sang total.

Des essais ont été effectués à partir de sang total. Une gamme de virus a été établie dans du sang total humain et du plasma de même origine, à quantité égale de latex. Les complexes latex/particules virales ont subi un traitement par la chaleur pour l'extraction des acides nucléiques. Les acides nucléiques libérés ont été amplifiés par RT-PCR à l'aide des amorces décrites précédemment.

Les résultats obtenus après passage sur gel d'agarose 2% (TBE + Gelstar) des produits d'amplification montrent une bande de taille attendue de 384 paires de bases à partir d'un titre viral de 5 10³ pfu dans le sang total et à partir de 50 pfu dans le plasma. Ces résultats montrent qu'il est possible d'isoler des particules virales à partir de sang total, bien que cela soit sensiblement moins performant qu'à partir de plasma. Les résultats sont résumés dans le tableau 3 ci dessous.

**Tableau 3**

| Echantillon | Titre infectieux en pfu | | | | | |
|---|---|---|---|---|---|---|
| | 0 | 10 | 50 | 500 | 5000 | 50 000 |
| Sang | - | - | - | - | + | + |
| Plasma | - | - | + | + | + | + |

### Exemple 9 : mise en culture de virus de la rougeole capturés.

Une gamme virale a été établie et testée à partir de plasma humain comme décrit dans l'exemple 8 (voir tableau 2). Les complexes latex magnétique/virus de la rougeole ont été mis en culture en présence de cellules Vero cultivées dans un milieu 199 contenant du glutamax (Gibco-BRL), 1% d'antibiotiques (GIBCO-BRL) et 10% de sérum de veau foetal. Les conditions de culture ont été établies comme décrites dans l'exemple 5. Une RT-PCR a été effectuée sur la gamme virale, le jour même de la mise en culture, pour valider la présence du virus dans l'échantillon mis en culture. Les cellules de la culture ont ensuite été observées sous microscope pour la mise en évidence d'un effet cytopathique induit par le virus infectieux dans la culture. Un effet cytopathique a été observé au bout du troisième jour pour les échantillons dont le titre infectieux était de 5 10⁴ et de 5 10³ pfu/ml et après 5 jours pour les échantillons dont le titre infectieux était de 500 et 50 pfu/ml.

### Exemple 10 : capture du virus du Syndrome de l'Immunodéficience Acquise (HIV-1) par le latex fonctionnalisé de l'invention, à partir d'un surnageant de culture, et étude de la viabilité virale.

### - Capture virale :

Une souche virale HIV-1 du groupe M, sous type G, a été cultivée en présence de PBMCs (cellules mononucléées du sang périphérique) dans un milieu de culture RPMI supplémenté par 10% de sérum de veau foetal. La présence du virus a été confirmée au 14^{ème} jour de culture par une détection de l'antigène P24 (31637,8 pg/ml, dilution au 1/100^{ème}) dans le surnageant de culture à l'aide du test immunoenzymatique VIDAS HIV P24 Il réalisé sur l'appareil VIDAS (marque enregistrée - bioMérieux). 150 µl de ce surnageant de culture, ont été mis en présence de 10 µl de latex magnétique, obtenu comme décrit dans l'exemple 1, pendant 10 minutes, à température ambiante et sous agitation. L'étape de capture a été suivie d'une étape d'aimantation pour la séparation du culot du surnageant de culture et le culot ainsi obtenu (complexe latex-virus) a été lavé par environ 100 µl de milieu de culture RPMI, puis aimanté de nouveau et resuspendu dans 10 µl de milieu RPMI.

### - Viabilité :

10 µl du complexe latex-virus préparé comme décrit ci-dessus ont été mis en présence d'un culot de PBMCs (25 10⁶ cellules), fraîchement préparées. L'ensemble a été homogénéisé, puis soumis à incubation à 37°C pendant deux heures pour l'étape d'infection des PBMCs, sous atmosphère de CO₂ (5%). 15 ml de milieu RPMI ont ensuite été ajoutés. L'ensemble a été homogénéisé et 5 ml de milieu ont été prélevés au jour J0 et soumis à centrifugation à 1100 tpm pendant 10 minutes. 1 ml de surnageant servira au dosage de la P24 et le reste est remis dans le milieu de culture correspondant. Parallèlement des PBMCs fraîchement préparées ont été mises en culture, dans les mêmes conditions, en présence de 10 µl de latex seul (sans virus) pour constituer le témoin négatif. Le dosage de l'antigène P24 est réalisé au moyen du test immunoenzymatique (VIDAS HIV P24 II) sur l'appareil VIDAS, dans le surnageant de culture témoin et infecté, respectivement aux jours J0, J2, J4, J7, J9, J11, J14, J16 et J18. Les résultats obtenus montrent une augmentation significative de l'antigène P24 dans le surnageant de culture dès le 14^{ème} jour de culture, ce qui confirme d'une part, que le virus a bien été capturé par le latex de l'invention et que d'autre part, il conserve, après capture, sa viabilité et son pouvoir réplicatif (infectiosité).

### Exemple 11 : capture du virus de l'hépatite B (HBV).

Des dilutions au 1/10 ont été effectuées à partir d'un sérum HBV positif dans un volume final de 150 µl contenant 10 000 à 10 copies virales. La capture du virus et l'extraction de l'ADN viral ont été faites selon le protocole décrit dans l'exemple 2.

La détection du nombre de copies virales a été réalisée par PCR nichée en utilisant des amorces définies dans la région S du génome HBV pour l'amplification d'un fragment de 440 paires de bases.
1^{er} tour: Amorce 1 : 5′ CCT GCT GGT GGC TCC AGT TC 3′ Amorce 2 : 5' TAC CCA AAG ACA AAA GAA AAT TGG 3'
2^{ème} tour:

Amorce 3 : 5′ TAG TAA ACT GAG CCA RGA GAA AC 3′

Amorce 4 : 5′ GTT GAC AAR AAT CCT CAC AAT AC 3′

Les produits d'amplification sont ensuite passés sur un gel d'agarose 1% et colorés au bromure d'éthidium. Un signal est détecté à la dilution 10⁻³ correspondant à un seuil de détection de 10 copies HBV, représentatif d'une bonne sensibilité du système de capture de l'invention.

Par ailleurs, un sérum humain positif a été dilué dans du sérum humain négatif de manière à obtenir 100 copies de HBV dans deux volumes différents de 100 et 1 000 µl, afin d'étudier un effet potentiel de concentration du latex de l'invention. Après capture des particules virales, contenues dans les deux échantillons dilués, par 10 µl de latex, les acides nucléiques ont été extraits par lyse par la chaleur et amplifiés par PCR nichée en utilisant les amorces décrites ci-dessus. Les produits d'amplification sont ensuite passés sur gel d'agarose 1 % et colorés au bromure d'éthidium. Les résultats montrent, pour les produits amplifiés à partir des deux échantillons dilués, une bande à la taille attendue de 440 paires de bases. L'intensité de coloration des deux fragments amplifiés, issus respectivement des deux échantillons dilués précités, se révèle être identique, ce qui prouve un effet de concentration du latex magnétique de l'invention.

## Revendications

1. Procédé d'isolement de protéines et/ou d'une association de protéines et d'acides nucléiques dans un échantillon **caractérisé en ce que** :
- on met en contact ledit échantillon et des particules colloïdales magnétiques comprenant un coeur et une enveloppe dans lesquelles :
le coeur est magnétique et enrobé par au moins un polymère comprenant des groupements fonctionnels X choisis parmi les groupements amine, hydroxyle, thiol, aldéhyde, ester, anhydride, chlorure d'acide, carbonate, carbamate, isocyanate et isothiocyanate ou leurs mélanges, dont au moins une fraction a réagi avec d'autres groupements fonctionnels de l'enveloppe, et
l'enveloppe est constituée d'un polymère portant des groupements fonctionnels ionisables, Z et Z', identiques ou différents, choisis parmi les groupements amine, acide carboxylique, ester, anhydride, aldéhyde, thiol, disulfure, α-halogénocarbonyle, acide sulfonique, maléimide, isocyanate, et isothiocyanate, qui ont partiellement réagi avec les groupements fonctionnels X du coeur ;
pour constituer un mélange,
- on incube ledit mélange sous des conditions prédéterminées, et
- on sépare du mélange les protéines et/ou les associations de protéines et d'acides nucléiques complexées sur lesdites particules colloïdales par application d'un champ magnétique.

2. Procédé selon la revendication 1, **caractérisé en ce que** le coeur comprend au moins un polymère organique choisi parmi au moins un homopolymère ou un copolymère ou leurs mélanges, issu de la polymérisation d'au moins un monomère choisi parmi les monomères d'acrylamide et d'acrytate, en particulier les N-alkylacrylamide et N, N-dialkylacrylamide, tels que le N-isopropylacrylamide, le N-méthylacrylamide, le N-éthylméthacrylamide, le N-n-propylacrylamide, le N-n-propylméthacrylamide, le N-isopropylméthacrylamide, le N-cyclopropylacrylamide, le N,N-diéthylacrylamide, le N-méthyl-N-isopropylacrylamide, le N-méthyl-N-n-propylacrylamide ; les acrylates et les méthacrylates d'alkyle dans lesquels le groupement alkyle comprend de 3 à 20 atomes de carbone ; le styrène, le méthylstyrène, l'éthylstyrène, le tertio-butyl-styrène, le chlorométhylstyrène vinyltoluène ; leurs dérivés et les copolymères. de ces monomères entre eux et/ou avec d'autres comonomères, et des particules d'oxydes métalliques choisies parmi les particules d'oxydes métalliques, de fer, de titane, de cobalt, de zinc, de cuivre, de manganèse, de nickel ; la magnétite ; l'hématite, les ferrites, telles que les ferrites de manganèse, nickel, manganèse-zinc; les alliages de cobalt, nickel.

3. Procédé selon la revendication 1, **caractérisé en ce que** le polymère d'enveloppe est choisi parmi au moins un homopolymère ou copolymère hydrophile choisi parmi les homopolymères ou copolymères :
- issus de la polymérisation d'au moins un monomère choisi parmi les monomères dérivés de l'acrylamide ou de méthacrylamide; l'acide acrylique, l'acide méthacrylique ; les dérivés acrylates et méthacrylates ; l'allylamine ; les dérivés styréniques ; à la condition s'il s'agit d'un homopolymère que celui ci comporte des groupements fonctionnels ionisables ; en particulier les copolymères ou homoplymères de l'anhydride maléique et les homopolymères ou copolymères de l'acryloxysuccinimide,
- les polysaccharides, tels que le chitosane et le polyacide galacturonique,
- les polypeptides, tels que la polylysine et la polyarginine,
- la polyéthylèneimine linéaire ou ramifiée, et
- les dendrimères.

4. Procédé selon la revendication 3, **caractérisé en ce que** le polymère d'enveloppe est le poly(anhydride maléique vinyl éther) le poly(N-vinylmorphofine-N-acryloxysuccinimide) ou le poly(N-vinylpyrrolidone-N-acryloxysuccinimide).

5. Procédé selon les revendications précédentes, **caractérisé en ce que** le mélange est soumis à incubation à une température comprise entre 15 et 60°C, de préférence entre 20 et 35°C, pendant un temps d'incubation compris entre 5 et 60 minutes, de préférence 10 minutes.

6. Procédé selon les revendications précédentes, **caractérisé en ce que** l'échantillon est un prélèvement ou un surnageant de culture comprenant des protéines et/ou des associations de protéines et d'acides nucléiques, en particulier un virus, une bactérie, une levure, une cellule, éventuellement en mélange.

7. Procédé selon la revendication 6, **caractérisé en ce que** l'échantillon est un échantillon biologique tel qu'un prélèvement tissulaire, de sang total, de plasma, de sérum ou un surnageant de culture, ou un prélèvement agroalimentaire.

8. Procédé d'extraction de protéines et/ou d'acides nucléiques, **caractérisé en ce que** :
- on isole d'un échantillon, par exemple d'un prélèvement ou d'un surnageant de culture, un virus et/ou une bactérie et/ou une levure et/ou, une cellule ou un mélange de ces derniers selon le procédé décrit dans les revendications 1 à 7 et,
- on soumet, si nécessaire, lesdits virus, bactérie, levure, cellule ou leur mélange à une étape de relargage et/ou de dénaturation partielle ou totale pour l'extraction desdites protéines et/ou desdits acides nucléiques.

9. Procédé de d'identification et/ou de détection et/ou de quantification de protéines et/ou d'une association de protéines et d'acides nucléiques et/ou d'acides nucléiques, **caractérisé en ce que** :
- on isole d'un échantillon, par exemple d'un prélèvement ou d'un surnageant de culture, un virus et/ou une bactérie et/ou une levure et/ou une cellule ou un mélange de ces derniers selon le procédé décrit dans les revendications 1 à 7 et,
- on soumet, si nécessaire, lesdits virus et/ou bactérie et/ou levure et/ou cellule ou leur mélange à une étape de relargage et/ou de dénaturation partielle ou totale pour l'extraction desdites protéines et/ou desdits acides nucléiques, et
- on identifie et/ou détecte et/ou quantifie lesdites protéines par immunodosage et/ou lesdits acides nucléiques par amplification de ces derniers et/ou par hybridation d'au moins une sonde nucléotidique spécifique desdits acides nucléiques à identifier et/ou détecter et/ou quantifier.

10. Procédé selon la revendication 9, **caractérisé en ce que** les protéines sont des protéines de surface ou intracellulaires d'un virus, d'une bactérie, d'une levure ou d'une cellule et **en ce que** les acides nucléiques sont de l'ADN et/ou de l'ARN.

11. Procédé selon les revendications 9 et 10, **caractérisé en ce que** les protéines sont identifiées et/ou détectées et/ou quantifiées (i) soit directement, sans étape de relargage et/ou de dénaturation, à partir desdits virus et/ou bactérie et/ou levure et/ou cellule isolés ou dans un surnageant de culture, (ii) soit indirectement, après une étape de relargage et/ou de dénaturation partielle ou totale réalisée, par exemple, par modification de pH.

12. Procédé selon les revendications 9 et 10, **caractérisé en ce que** les acides nucléiques sont identifiés et/ou détectés et/ou quantifiés après une étape de relargage et/ou de dénaturation partielle ou totale desdits virus et/ou bactérie et/ou levure et/ou cellule isolés réalisée, par exemple, par un traitement chimique et/ou physique.

13. Procédé pour la mise en culture d'un virus et/ou d'une bactérie, et/ou d'une levure et/ou ou de cellules, **caractérisé en ce que** :
- on isole à partir d'un échantillon, ledit virus et/ou ladite bactérie et/ou ladite levure et/ou lesdites cellules selon le procédé décrit dans les revendications 1 à 7,
- on met en culture, dans un milieu de culture et sous des conditions appropriés, lesdits virus et/ou bactérie et/ou levure et/ou cellules ainsi isolés, et si souhaité,
- on identifie et/ou détecte et/ou quantifie lesdits virus et/ou bactérie et/ou levure et/ou cellules.

14. Procédé de préparation d'un échantillon biologique, **caractérisé en ce que** :
- on isole de l'échantillon des protéines et/ou des associations de protéines et d'acides nucléiques selon le procédé décrit dans les revendications 1 à 7, et/ou
- on réalise une culture comme décrit dans la revendication 13.

15. Complexe **caractérisé en ce qu'**il est formé de particules colloïdales magnétiques et de protéines et/ou d'une association de protéines et d'acides nucléiques, lesdites particules colloïdales comprenant un coeur et une enveloppe dans lesquelles :
le coeur est magnétique et enrobé par au moins un polymère comprenant des groupements fonctionnels X choisis parmi les groupements amine, hydroxyle, thiol, aldéhyde, ester, anhydride, chlorure d'acide , carbonate, carbamate, isocyanate et isothiocyanate ou leurs mélanges, dont au moins une fraction a réagi avec d'autres groupements fonctionnels de l'enveloppe, et
l'enveloppe est constituée d'un polymère portant des groupements fonctionnels ionisables, Z et Z', identiques ou différents, choisis parmi les groupements amine, acide carboxylique, ester, anhydride, aldéhyde, thiol, disulfure, α-halogénocarbonyle, acide sulfonique, maléimide, isocyanate, et isothiocyanate, qui ont partiellement réagi avec les groupements fonctionnels X du coeur; et
lesdites protéines et/ou associations de protéines et d'acides nucléiques étant issues d'un échantillon biologique, par exemple d'un prélèvement, tel qu'un prélèvement tissulaire, de sang total, de sérum, ou d'un surnageant de culture, ou d'un prélèvement agroalimentaire.

16. Complexe selon la revendication 15, **caractérisé en ce que** le prélèvement ou le surnageant de culture sont infectés par au moins un virus, une bactérie, une levure ou leur mélange.

17. Complexe selon les revendications 15 et 16, **caractérisé en ce que** le coeur comprend au moins un polymère organique choisi parmi au moins un homopolymère ou un copolymère ou leurs mélanges, issu de la polymérisation d'au moins un monomère choisi parmi les monomères d'acrylamide et d'acrylate, en particulier les N-alkylacrylamide et N-N-dialkylacrylamide, tels que le N-isopropylacrylamide, le N-méthylacrylamide, le N-éthylméthacrylamide, le N-n-propylacrylarnide, le N-n-propylméthacrylamide, le N-isopropylméthacrylamide, le N-cyclopropylacrylamide, le N, N-diéthylacrylamide, le N-méthyl-N-isopropylacrylamide, le N-méthyl-N-n-propylacrylamide ; les acrylates et les méthacrylates d'alkyle dans lesquels le groupement alkyle comprend de 3 à 20 atomes de carbone ; le styrène, le méthylstyrène, l'éthylstyrène, le tertio-butyl-styrène, le chlorométhylstyrène vinyltoluène ; leurs dérivés et les copolymères de ces monomères entre eux et/ou avec d'autres comonomères, et des particules d'oxydes métalliques choisies parmi les particules d'oxydes métalliques, de fer, de titane, de cobalt, de zinc, de cuivre, de manganèse, de nickel ; la magnétite ; l'hématite, les ferrites, telles que les ferrites de manganèse, nickel, manganèse-zinc; les alliages de cobalt, nickel.

18. Complexe selon les revendications 15 et 16, **caractérisé en ce que** le polymère de l'enveloppe est choisi parmi au moins un homopolymère ou copolymère hydrophile choisi parmi les homopolymères ou copolymères :
- issus de la polymérisation d'au moins un monomère choisi parmi les monomères dérivés de l'acrylamide ou de méthacrylamide ; l'acide acrylique, l'acide méthacrylique ; les dérivés acrylates et méthacrylates ; l'allylamine ; les dérivés styréniques ; à la condition s'il s'agit d'un homopolymère que celui ci comporte des groupements fonctionnels ionisables ; en particulier les copolymères ou homoplymères de l'anhydride maléique et les homopolymères ou copolymères de l'acryloxysuccinimide,
- les polysaccharides, tels que le chitosane et le polyacide galacturonique,
- les polypeptides, tels que la polylysine et la polyarginine,
- la polyéthylèneimine linéaire ou ramifiée, et
- les dendrimères.

19. Complexe selon la revendication 18, **caractérisé en ce que** le polymère d'enveloppe est le poly(anhydride maléique vinyl éther) le poly(N-vinylmorpholine-N-acryloxysuccinimide) ou le poly(N-vinylpyrrolidone-N-acryloxysuccinimide).

20. Utilisation d'un complexe tel que défini dans les revendications 15 à 19, pour le transfert et/ou le transport et/ou le stockage d'agents infectieux, en particulier de virus et/ou bactérie et/ou levure.

21. Réactif pour l'extraction et/ou l'identification et/ou la détection et/ou la quantification de protéines et/ou d'associations de protéines et d'acides nucléiques, **caractérisé en ce qu'**il comprend, entre autres, un complexe tel que défini dans les revendications 15 à 19.

22. Réactif pour l'extraction et/ou l'identification et/ou la détection et/ou la quantification de protéines et/ou d'associations de protéines et d'acides nucléiques, **caractérisé en ce qu'**il comprend des particules colloïdales définies à l'une quelconque des revendications 1 à 4, permettant d'obtenir un complexe tel que défini à l'une des revendications 15 et 16.

23. Réactif selon la revendication 21 ou 22, **caractérisé en ce qu'**il comprend de plus au moins un moyen pour l'identification et/ou la détection et/ou la quantification desdites protéines ou desdits acides nucléiques, en particulier au moins un anticorps monoclonal ou polyclonal pour l'identification et/ou la détection et/ou la quantification d'au moins une protéine, au moins une amorce, de préférence au moins deux amorces et/ou au moins une sonde nucléotidique, de préférence au moins deux sondes nucléotidiques spécifiques d'au moins un acide nucléique pour son identification et/ou sa détection et/ou sa quantification.

24. Composition vaccinale, **caractérisée en ce qu'**elle comprend, à titre de principe actif, au moins un complexe selon les revendications 15 à 19, éventuellement associé à un véhicule et/ou un excipient et/ou un adjuvant et/ou un diluant pharmaceutiquement acceptable.

25. Véhicule pharmaceutiquement acceptable pour composition vaccinale, **caractérisé en ce qu'**il consiste en une particule comprenant un coeur et une enveloppe telle que définie à l'une quelconque des revendications 1 à 4.

## Patentansprüche

1. Verfahren zur Isolation von Proteinen und/oder einer Assoziation von Proteinen und Nucleinsäuren in einer Probe, **dadurch gekennzeichnet, dass**:
- die Probe mit kolloidalen magnetischen Partikeln in Kontakt gebracht wird, die einen Kern und eine Hülle umfassen, worin:
der Kern magnetisch ist und durch mindestens ein Polymer eingehüllt wird, das funktionelle Gruppen X umfasst, die aus den Amin-, Hydroxyl-, Thiol-, Aldehyd-, Ester-, Anhydrid-, Säurechlorid-, Carbonat-, Carbamat-, Isocyanat- und Isothiocyanat-Gruppen oder deren Gemischen ausgewählt sind, von denen mindestens eine Fraktion mit anderen funktionellen Gruppen der Hülle reagiert hat, und
die Hülle aus einem Polymer besteht, das ionisierbare funktionelle Gruppen Z und Z' trägt, die gleich oder verschieden sind und aus den Amin-, Carbonsäure-, Ester-, Anhydrid-, Aldehyd-, Thiol-, Disulfid-, α-Halogencarbonyl-, Sulfonsäure-, Maleinimid-, Isocyanat- und Isothiocyanat-Gruppen ausgewählt sind, die teilweise mit den funktionellen Gruppen X des Kerns reagiert haben,
um ein Gemisch herzustellen,
- das Gemisch unter vorbestimmten Bedingungen inkubiert wird und
- die Proteine und/oder die Assoziationen von Proteinen und Nucleinsäuren, die auf den kolloidalen Partikeln komplexiert sind, durch Anlegen eines Magnetfelds von dem Gemisch abgetrennt werden.

2. Verfahren nach Anspruch 1, **dadurch gekennzeichnet, dass** der Kern mindestens ein organisches Polymer, das aus mindestens einem Homopolymer oder einem Copolymer oder ihren Gemischen ausgewählt ist, die aus der Polymerisation von mindestens einem Monomer, das aus Acrylamid- und Acrylatmonomeren, insbesondere N-Alkylacrylamid und N,N-Dialkylacrylamid, wie N-Isopropylacrylamid, N-Methylacrylamid, N-Ethylmethacrylamid, N-n-Propylacrylamid, N-n-Propylmethacrylamid, N-Isopropylmethacrylamid, N-Cyclopropylacrylamid, N,N-Diethylacrylamid, N-Methyl-N-isopropylacrylamid, N-Methyl-N-n-propylacrylamid; Alkylacrylaten und -methacrylaten, in denen der Alkylrest 3 bis 20 Kohlenstoffatome umfasst; Styrol, Methylstyrol, Ethylstyrol, tert.-Butylstyrol, Chlormethylstyrolvinyltoluol; ihren Derivaten und den Copolymeren dieser Monomere untereinander und/oder mit anderen Comonomeren ausgewählt ist, stammen, und Metalloxidpartikel umfasst, die aus den Partikeln von Metalloxiden von Eisen, Titan, Kobalt, Zink, Kupfer, Mangan, Nickel; Magnetit; Hämatit, Ferriten, wie Ferriten von Mangan, Nickel, Mangan-Zink; Kobalt-Nickel-Legierungen ausgewählt sind.

3. Verfahren nach Anspruch 1, **dadurch gekennzeichnet, dass** das Polymer der Hülle aus mindestens einem hydrophilen Homopolymer oder Copolymer ausgewählt ist, das aus den folgenden Homopolymeren oder Copolymeren ausgewählt ist:
- aus den aus der Polymerisation von mindestens einem Monomer hervorgehenden, das aus den von Acrylamid oder Methacrylamid stammenden Monomeren; Acrylsäure, Methacrylsäure; Acrylat- und Methacrylat-Derivaten; Allylamin; Styrolderivaten ausgewählt ist, mit der Maßgabe, dass, wenn es sich um ein Homopolymer handelt, dieses ionisierbare funktionelle Gruppen trägt, insbesondere die Copolymere oder Homopolymere von Maleinsäureanhydrid und die Homopolymere oder Copolymere von Acryloxysuccinimid,
- aus Polysacchariden, wie Chitosan und Polygalacturonsäure,
- Polypeptiden, wie Polylysin und Polyarginin,
- linearem oder verzweigtem Polyethylenimin und
- Dendrimeren.

4. Verfahren nach Anspruch 3, **dadurch gekennzeichnet, dass** das Polymer der Hülle Poly(maleinsäureanhydridvinylether), Poly(N-Vinylmorpholin-N-acryloxysuccinimid) oder Poly(N-Vinylpyrrolidin-N-acryloxysuccinimid) ist.

5. Verfahren nach einem der vorstehenden Ansprüche, **dadurch gekennzeichnet, dass** das Gemisch einer Inkubation bei einer Temperatur zwischen 15 und 60°C, vorzugsweise zwischen 20 und 35°C, für eine Inkubationszeit zwischen 5 und 60 Minuten, vorzugsweise von 10 Minuten, unterworfen wird.

6. Verfahren nach einem der vorstehenden Ansprüche, **dadurch gekennzeichnet, dass** die Probe eine Entnahme oder ein Kulturüberstand ist, der Proteine und/oder Assoziationen von Proteinen und Nucleinsäuren, insbesondere ein Virus, eine Bakterie, eine Hefe, eine Zelle, gegebenenfalls in einer Mischung, umfasst.

7. Verfahren nach Anspruch 6, **dadurch gekennzeichnet, dass** die Probe eine biologische Probe, wie eine Gewebeentnahme, Gesamtblut, Plasma, Serum oder ein Kulturüberstand, oder eine Agronahrungsmittelentnahme ist.

8. Verfahren zur Extraktion von Proteinen und/oder Nucleinsäuren, **dadurch gekennzeichnet, dass**:
- aus einer Probe, beispielsweise einer Entnahme oder einem Kulturüberstand, ein Virus und/oder eine Bakterie und/oder eine Hefe und/oder eine Zelle und/oder eine Mischung von diesen gemäß dem in den Ansprüchen 1 bis 7 beschriebenen Verfahren isoliert wird und,
- wenn nötig, das Virus, die Bakterie, die Hefe, die Zelle oder ihr Gemisch einem Schritt der Aussalzung und/oder der partiellen oder vollständigen Denaturierung zur Extraktion der Proteine und/oder der Nucleinsäuren unterzogen wird.

9. Verfahren zur Identifikation und/oder zum Nachweis und/oder zur Quantifizierung von Proteinen und/oder einer Assoziation von Proteinen und Nucleinsäuren und/oder Nucleinsäuren, **dadurch gekennzeichnet, dass**:
- aus einer Probe, beispielsweise einer Entnahme oder einem Kulturüberstand, ein Virus und/oder eine Bakterie und/oder eine Hefe und/oder eine Zelle und/oder eine Mischung von diesen gemäß dem in den Ansprüchen 1 bis 7 beschriebenen Verfahren isoliert wird und,
- wenn nötig, das Virus und/oder die Bakterie und/oder die Hefe und/oder die Zelle oder ihr Gemisch einem Schritt der Aussalzung und/oder der partiellen oder vollständigen Denaturierung zur Extraktion der Proteine und/oder der Nucleinsäuren unterzogen wird und
- die Proteine durch Immunbestimmung und/oder die Nucleinsäuren durch ihre Amplifikation und/oder durch Hybridisierung mit mindestens einer für die zu identifizierenden und/oder nachzuweisenden und/oder zu quantifizierenden Nucleinsäuren spezifischen Nucleotidsonde identifiziert und/oder nachgewiesen und/oder quantifiziert werden.

10. Verfahren nach Anspruch 9, **dadurch gekennzeichnet, dass** die Proteine Oberflächenproteine oder intrazelluläre Proteine eines Virus, einer Bakterie, einer Hefe oder einer Zelle sind, und dadurch, dass die Nucleinsäuren DNA und/oder RNA sind.

11. Verfahren nach den Ansprüchen 9 und 10, **dadurch gekennzeichnet, dass** die Proteine (i) direkt, ohne Aussalzungs- und/oder Denaturierungsschritt, ausgehend von dem isolierten Virus und/oder der isolierten Bakterie und/oder der isolierten Hefe und/oder der isolierten Zelle oder in einem Kulturüberstand, (ii) indirekt, nach einem Aussalzungs- und/oder partiellen oder vollständigen Denaturierungsschritt, der beispielsweise durch Modifikation des pH durchgeführt wird, identifiziert und/oder nachgewiesen und/oder quantifiziert werden.

12. Verfahren nach den Ansprüchen 9 und 10, **dadurch gekennzeichnet, dass** die Nucleinsäuren nach einem Aussalzungs- und/oder partiellen oder vollständigen Denaturierungsschritt des isolierten Virus und/oder der isolierten Bakterie und/oder der isolierten Hefe und/oder der isolierten Zelle, der beispielsweise durch eine chemische und/oder physikalische Behandlung durchgeführt wird, identifiziert und/oder nachgewiesen und/oder quantifiziert werden.

13. Verfahren zur Durchführung einer Kultur eines Virus und/oder einer Bakterie und/oder einer Hefe und/oder von Zellen, **dadurch gekennzeichnet, dass**:
- ausgehend von einer Probe das Virus und/oder die Bakterie und/oder die Hefe und/oder die Zellen gemäß dem in den Ansprüchen 1 bis 7 beschriebenen Verfahren isoliert werden,
das so isolierte Virus und/oder die so isolierte Bakterie und/oder die so isolierte Hefe und/oder die so isolierten Zellen in einem Kulturmedium und unter geeigneten Bedingungen in Kultur gebracht werden und, wenn gewünscht,
- das Virus und/oder die Bakterie und/oder die Hefe und/oder die Zellen identifiziert und/oder nachgewiesen und/oder quantifiziert werden.

14. Verfahren zur Herstellung einer biologischen Probe, **dadurch gekennzeichnet, dass**:
- aus der Probe die Proteine und/oder die Assoziationen von Proteinen und Nucleinsäuren gemäß dem in den Ansprüchen 1 bis 7 beschriebenen Verfahren isoliert werden und/oder
- eine Kultur, wie im Anspruch 13 beschrieben, durchgeführt wird.

15. Komplex, **dadurch gekennzeichnet, dass** er von kolloidalen magnetischen Partikeln und Proteinen und/oder einer Assoziation von Proteinen und Nucleinsäuren gebildet wird, wobei die kolloidalen Partikel einen Kern und eine Hülle umfassen, worin:
der Kern magnetisch ist und durch mindestens ein Polymer eingehüllt wird, das funktionelle Gruppen X umfasst, die aus den Amin-, Hydroxyl-, Thiol-, Aldehyd-, Ester-, Anhydrid-, Säurechlorid-, Carbonat-, Carbamat-, Isocyanat- und Isothiocyanat-Gruppen oder deren Gemischen ausgewählt sind, von denen mindestens eine Fraktion mit anderen funktionellen Gruppen der Hülle reagiert hat, und
die Hülle aus einem Polymer besteht, das ionisierbare funktionelle Gruppen Z und Z' trägt, die gleich oder verschieden sind und aus den Amin-, Carbonsäure-, Ester-, Anhydrid-, Aldehyd-, Thiol-, Disulfid-, α-Halogencarbonyl-, Sulfonsäure-, Maleinimid-, Isocyanat- und Isothiocyanat-Gruppen ausgewählt sind, die teilweise mit den funktionellen Gruppen X des Kerns reagiert haben; und
- die Proteine und/oder die Assoziationen von Proteinen und Nucleinsäuren aus einer biologischen Probe, beispielsweise einer Entnahme, wie einer Gewebeentnahme, Gesamtblut, Serum oder einem Kulturüberstand, oder aus einer Agronahrungsmittelentnahme stammen.

16. Komplex nach Anspruch 15, **dadurch gekennzeichnet, dass** die Entnahme oder der Kulturüberstand durch mindestens ein Virus, eine Bakterie, eine Hefe oder deren Gemisch infiziert sind.

17. Komplex nach den Ansprüchen 15 und 16, **dadurch gekennzeichnet, dass** der Kern mindestens ein organisches Polymer, das aus mindestens einem Homopolymer oder einem Copolymer oder ihren Gemischen ausgewählt ist, die aus der Polymerisation von mindestens einem Monomer, das aus Acrylamid- und Acrylatmonomeren, insbesondere N-Alkylacrylamid und N,N-Dialkylacrylamid, wie N-Isopropylacrylamid, N-Methylacrylamid, N-Ethylmethacrylamid, N-n-Propylacrylamid, N-n-Propylmethacrylamid, N-Isopropylmethacrylamid, N-Cyclopropylacrylamid, N,N-Diethylacrylamid, N-Methyl-N-isopropylacrylamid, N-Methyl-N-n-propylacrylamid; Alkylacrylaten und -methacrylaten, in denen der Alkylrest 3 bis 20 Kohlenstoffatome umfasst; Styrol, Methylstyrol, Ethylstyrol, tert.-Butylstyrol, Chlormethylstyrolvinyltoluol; ihren Derivaten und den Copolymeren dieser Monomere untereinander und/oder mit anderen Comonomeren ausgewählt ist, stammen, und Metalloxidpartikel umfasst, die aus den Partikeln von Metalloxiden von Eisen, Titan, Kobalt, Zink, Kupfer, Mangan, Nickel; Magnetit; Hämatit, Ferriten, wie Ferriten von Mangan, Nickel, Mangan-Zink; Kobalt-Nickel-Legierungen ausgewählt sind.

18. Komplex nach den Ansprüchen 15 und 16, **dadurch gekennzeichnet, dass** das Polymer der Hülle aus mindestens einem hydrophilen Homopolymer oder Copolymer ausgewählt ist, das aus den folgenden Homopolymeren oder Copolymeren ausgewählt ist:
- aus den aus der Polymerisation von mindestens einem Monomer hervorgehenden, das aus den von Acrylamid oder Methacrylamid stammenden Monomeren; Acrylsäure, Methacrylsäure; Acrylat- und Methacrylat-Derivaten; Allylamin; Styrolderivaten ausgewählt ist, mit der Maßgabe, dass, wenn es sich um ein Homopolymer handelt, dieses ionisierbare funktionelle Gruppen trägt, insbesondere die Copolymere oder Homopolymere von Maleinsäureanhydrid und die Homopolymere oder Copolymere von Acryloxysuccinimid,
- aus Polysacchariden, wie Chitosan und Polygalacturonsäure,
- Polypeptiden, wie Polylysin und Polyarginin,
- linearem oder verzweigtem Polyethylenimin und
- Dendrimeren.

19. Komplex nach Anspruch 18, **dadurch gekennzeichnet, dass** das Polymer der Hülle Poly(maleinsäureanhydridvinylether), Poly(N-Vinylmorpholin-N-acryloxysuccinimid) oder Poly(N-Vinylpyrrolidin-N-acryloxysuccinimid) ist.

20. Verwendung eines Komplexes nach den Ansprüchen 15 bis 19 zur Überführung und/oder zum Transport und/oder zur Aufbewahrung von Infektionserregern, insbesondere einem Virus und/oder einer Bakterie und/oder einer Hefe.

21. Reagenz zur Extraktion und/oder Identifikation und/oder zum Nachweis und/oder zur Quantifizierung von Proteinen und/oder Assoziationen von Proteinen und Nucleinsäuren, **dadurch gekennzeichnet, dass** es unter anderem einen Komplex, wie in den Ansprüchen 15 bis 19 definiert, umfasst.

22. Reagenz zur Extraktion und/oder Identifikation und/oder zum Nachweis und/oder zur Quantifizierung von Proteinen und/oder Assoziationen von Proteinen und Nucleinsäuren, **dadurch gekennzeichnet, dass** es kolloidale Partikel, wie in einem der Ansprüche 1 bis 4 definiert, umfasst, welche den Erhalt eines Komplexes, wie in einem der Ansprüche 15 und 16 definiert, gestatten.

23. Reagenz nach Anspruch 21 oder 22, **dadurch gekennzeichnet, dass** es außerdem mindestens ein Mittel zur Identifikation und/oder zum Nachweis und/oder zur Quantifizierung der Proteine oder Nucleinsäuren umfasst, insbesondere mindestens einen monoklonalen oder polyklonalen Antikörper zur Identifikation und/oder zum Nachweis und/oder zur Quantifizierung von mindestens einem Protein, mindestens einen Primer, vorzugsweise mindestens zwei Primer und/oder mindestens eine Nucleotidsonde, vorzugsweise mindestens zwei Nucleotidsonden, die für mindestens eine Nucleinsäure spezifisch sind, für deren Identifikation und/oder Nachweis und/oder Quantifizierung.

24. Impfstoffzusammensetzung, **dadurch gekennzeichnet, dass** sie als Wirkstoff mindestens einen Komplex nach den Ansprüchen 15 bis 19, gegebenenfalls in Verbindung mit einem Vehikel und/oder einem Excipienten und/oder einem Adjuvans und/oder einem pharmazeutisch annehmbaren Verdünnungsmittel umfasst.

25. Vehikel, das für eine Impfstoffzusammensetzung pharmazeutisch annehmbar ist, **dadurch gekennzeichnet, dass** es aus einem Partikel besteht, das einen Kern und eine Hülle, wie in einem der Ansprüche 1 bis 4 definiert, umfasst.

## Claims

1. Method for isolating proteins and/or a protein and nucleic acid association, from a sample, **characterized in that**:
- said sample is brought into contact with magnetic colloidal particles comprising a core and an envelope in which:
the core is magnetic and is coated with at least one polymer comprising functional groups X chosen from amine, hydroxyl, thiol, aldehyde, ester, anhydride, acid chloride, carbonate, carbamate, isocyanate and isothiocyanate groups, or mixtures thereof, at least one fraction of which has reacted with other functional groups of the envelope, and
the envelope consists of a polymer bearing ionizable functional groups, Z and Z', which may be identical or different, chosen from amine, carboxylic acid, ester, anhydride, aldehyde, thiol, disulphide, α-halocarbonyl; sulphonic acid, maleimide, isocyanate and isothiocyanate groups, which have partially reacted with the functional groups X of the core;
so as to constitute a mixture,
- said mixture is incubated under predetermined conditions, and
- the proteins and/or the protein and nucleic acid associations complexed on said colloidal particles are separated from the mixture by applying a magnetic field.

2. Method according to Claim 1, **characterized in that** the core comprises at least one organic polymer chosen from at least one homopolymer or one copolymer, or mixtures thereof, derived from the polymerization of at least one monomer chosen from monomers of acrylamide and of acrylate, in particular N-alkylacrylamides and N,N-dialkylacrylamides, such as N-isopropylacrylamide, N-methylacrylamide, N-ethylmethacrylamide, N-n-propylacrylamide, N-n-propylmethacrylamide, N-isopropylmethacrylamide, N-cyclopropylacrylamide, N,N-diethylacrylamide, N-methyl-N-isopropylacrylamide or N-methyl-N-n-propylacrylamide; alkylacrylates and alkylmethacrylates in which the alkyl group comprises from 3 to 20 carbon atoms; styrene, methylstyrene, ethylstyrene, tert-butylstyrene, chloromethylstyrene vinyltoluene; derivatives thereof and the copolymers of these monomers with one another and/or with other comonomers, and metal oxide particles chosen from particles of metal oxides of iron, titanium, cobalt, zinc, copper, manganese, nickel; magnetite; hematite, ferrites such as manganese, nickel or manganese-zinc ferrites; alloys of cobalt, nickel.

3. Method according to Claim 1, **characterized in that** the envelope polymer is chosen from at least one hydrophilic homopolymer or copolymer chosen from homopolymers or copolymers:
- derived from the polymerization of at least one monomer chosen from monomers derived from acrylamide or from methacrylamide; acrylic acid, methacrylic acid; acrylate and methacrylate derivatives; allylamine; styrene derivatives; on the condition, if it is a homopolymer, that this homopolymer comprises ionizable functional groups; in particular copolymers or homopolymers of maleic anhydride and homopolymers or copolymers of acryloxysuccinimide,
- polysaccharides, such as chitosan and poly(galacturonic acid),
- polypeptides, such as polylysine and polyarginine,
- linear or branched polyethyleneimine, and
- dendrimers.

4. Method according to Claim 3, **characterized in that** the envelope polymer is poly(maleic anhydride vinyl ether), poly(N-vinylmorpholine-N-acryloxy-succinimide) or poly(N-vinylpyrrolidone-N-acryloxy-succinimide).

5. Method according to any one of the preceding claims, **characterized in that** the mixture is subjected to incubation at a temperature of between 15 and 60°C, preferably between 20 and 35°C, for an incubation time of between 5 and 60 minutes, preferably 10 minutes.

6. Method according to any one of the preceding claims, **characterized in that** the sample is a specimen or a culture supernatant comprising proteins and/or protein and nucleic acid associations, in particular a virus, a bacterium, a yeast and/or a cell, optionally as a mixture.

7. Method according to Claim 6, **characterized in that** the sample is a biological sample, such as a tissue specimen, a specimen of whole blood, of plasma or of serum or a culture supernatant, or an agrofoods specimen.

8. Method for extracting proteins and/or nucleic acids, **characterized in that**:
- a virus and/or a bacterium and/or a yeast and/or a cell or a mixture thereof is isolated from a sample, for example from a specimen or from a culture supernatant, according to the method described in Claims 1 to 7, and
- if necessary, said virus, bacterium, yeast, cell or mixture thereof is subjected to a step of partial or total release and/or denaturation for the extraction of said proteins and/or said nucleic acids.

9. Method for identifying and/or detecting and/or quantifying proteins and/or a protein and nucleic acid association and/or nucleic acids, **characterized in that**:
- a virus and/or a bacterium and/or a yeast and/or a cell or a mixture thereof is isolated from a sample, for example from a specimen or from a culture supernatant, according to the method described in Claims 1 to 7, and
- if necessary, said virus and/or bacterium and/or yeast and/or cell or mixture thereof is subjected to a step of partial or total release and/or denaturation for the extraction of said proteins and/or said nucleic acids, and
- said proteins are identified and/or detected and/or quantified by immunoassay and/or said nucleic acids are identified and/or detected and/or quantified by amplifying them and/or by hybridization of at least one nucleotide probe specific for said nucleic acids to be identified and/or detected and/or quantified.

10. Method according to Claim 9, **characterized in that** the proteins are surface or intracellular proteins of a virus, of a bacterium, of a yeast or of a cell, and **in that** the nucleic acids are DNA and/or RNA.

11. Method according to Claims 9 and 10, **characterized in that** the proteins are identified and/or detected and/or quantified (i) either directly, without a step of release and/or denaturation, from said virus and/or bacterium and/or yeast and/or cell, isolated or in a culture supernatant, (ii) or indirectly, after a step of partial or total release and/or denaturation carried out, for example, by modification of pH.

12. Method according to Claims 9 and 10, **characterized in that** the nucleic acids are identified and/or detected and/or quantified after a step of partial or total release and/or denaturation of said isolated virus and/or bacterium and/or yeast and/or cell carried out, for example, by chemical and/or physical treatment.

13. Method for culturing a virus and/or a bacterium and/or a yeast and/or cells, **characterized in that**:
- said virus and/or said bacterium and/or said yeast and/or said cells are isolated from a sample, according to the method described in Claims 1 to 7,
- said virus and/or bacterium and/or yeast and/or cells thus isolated are placed in culture, in a culture medium and under conditions which are suitable, and, if desired,
- said virus and/or bacterium and/or yeast and/or cells are identified and/or detected and/or quantified.

14. Method for preparing a biological sample, **characterized in that**:
- proteins and/or protein and nucleic acid associations are isolated from the sample, according to the method described in Claims 1 to 7, and/or
- a culture is prepared as described in Claim 13.

15. Complex, **characterized in that** it is made up of magnetic colloidal particles and proteins and/or a protein and nucleic acid association, said colloidal particles comprising a core and an envelope in which:
the core is magnetic and is coated with at least one polymer comprising functional groups X chosen from amine, hydroxyl, thiol, aldehyde, ester, anhydride, acid chloride, carbonate, carbamate, isocyanate and isothiocyanate groups, or mixtures thereof, at least one fraction of which has reacted with other functional groups of the envelope, and
the envelope consists of a polymer bearing ionizable functional groups, Z and Z', which may be identical or different, chosen from amine, carboxylic acid, ester, anhydride, aldehyde, thiol, disulphide, α-halocarbonyl, sulphonic acid, maleimide, isocyanate and isothiocyanate groups, some of which have reacted with the functional groups X of the core; and
said proteins and/or protein and nucleic acid associations being derived from a biological sample, for example from a specimen, such as a tissue specimen or a specimen of whole blood or of serum, or from a culture supernatant, or from an agrofoods specimen.

16. Complex according to Claim 15, **characterized in that** the specimen or the culture supernatant is infected with at least one virus, one bacterium or one yeast, or a mixture thereof.

17. Complex according to Claims 15 and 16, **characterized in that** the core comprises at least one organic polymer chosen from at least one homopolymer or one copolymer, or mixtures thereof, derived from the polymerization of at least one monomer chosen from monomers of acrylamide and of acrylate, in particular N-alkylacrylamides and N,N-dialkylacrylamides, such as N-isopropylacrylamide, N-methylacrylamide, N-ethylmethacrylamide, N-n-propylacrylamide, N-n-propylmethacrylamide, N-isopropylmethacrylamide, N-cyclopropylacrylamide, N,N-diethylacrylamide, N-methyl-N-isopropylacrylamide or N-methyl-N-n-propylacrylamide; alkylacrylates and alkylmethacrylates in which the alkyl group comprises from 3 to 20 carbon atoms; styrene, methylstyrene, ethylstyrene, tert-butyl-styrene, chloromethylstyrene vinyltoluene; derivatives thereof and the copolymers of these monomers with one another and/or with other comonomers, and metal oxide particles chosen from particles of metal oxides of iron, titanium, cobalt, zinc, copper, manganese, nickel; magnetite; hematite, ferrites such as manganese, nickel or manganese-zinc ferrites; alloys of cobalt, nickel.

18. Complex according to Claims 15 and 16, **characterized in that** the envelope polymer is chosen from at least one hydrophilic homopolymer or copolymer chosen from homopolymers or copolymers:
- derived from the polymerization of at least one monomer chosen from monomers derived from acrylamide or from methacrylamide; acrylic acid, methacrylic acid; acrylate and methacrylate derivatives; allylamine; styrene derivatives; on the condition, if it is a homopolymer, that this homopolymer comprises ionizable functional groups; in particular copolymers or homopolymers of maleic anhydride and homopolymers or copolymers of acryloxysuccinimide,
- polysaccharides, such as chitosan and poly(galacturonic acid),
- polypeptides, such as polylysine and polyarginine,
- linear or branched polyethyleneimine, and
- dendrimers.

19. Complex according to Claim 18, **characterized in that** the envelope polymer is poly(maleic anhydride vinyl ether), poly(N-vinylmorpholine-N-acryloxy-succinimide) or poly(N-vinylpyrrolidone-N-acryloxy-succinimide).

20. Use of a complex as defined in Claims 15 to 19, for the transfer and/or transport and/or storage of infectious agents, in particular of a virus and/or bacterium and/or yeast.

21. Reagent for extracting and/or identifying and/or detecting and/or quantifying proteins and/or protein and nucleic acid associations, **characterized in that** it comprises, inter alia, a complex as defined in Claims 15 to 19.

22. Reagent for extracting and/or identifying and/or detecting and/or quantifying proteins and/or protein and nucleic acid associations, **characterized in that** it comprises colloidal particles defined in any one of Claims 1 to 4, which make it possible to obtain a complex as defined in either of Claims 15 and 16.

23. Reagent according to Claim 21 or 22, **characterized in that** it also comprises at least one means for identifying and/or detecting and/or quantifying said proteins or said nucleic acids, in particular at least one monoclonal or polyclonal antibody for identifying and/or detecting and/or quantifying at least one protein, and at least one primer, preferably at least two primers, and/or at least one nucleotide probe, preferably at least two nucleotide probes, specific for at least one nucleic acid, for the identification and/or detection and/or quantification thereof.

24. Vaccinal composition, **characterized in that** it comprises, as active principle, at least one complex according to Claims 15 to 19, optionally combined with a pharmaceutically acceptable vehicle and/or excipient and/or adjuvant and/or diluent.

25. Pharmaceutically acceptable vehicle for a vaccinal composition, **characterized in that** it consists of a particle comprising a core and an envelope as defined in any one of Claims 1 to 4.
